Europäisches Patentamt

European Patent Office

Office européen des brevets

(11) Veröffentlichungsnummer: **0 465 841 A2**

# EUROPÄISCHE PATENTANMELDUNG

(21) Anmeldenummer: **91109392.0**

(22) Anmeldetag: **07.06.91**

(51) Int. Cl.5: **A61K 9/72**, A61K 9/12, A61K 9/14

(30) Priorität: **13.06.90 DE 4018861**

(43) Veröffentlichungstag der Anmeldung: **15.01.92 Patentblatt 92/03**

(84) Benannte Vertragsstaaten: **AT BE CH DE DK ES FR GB GR IT LI LU NL SE**

(71) Anmelder: **HOECHST AKTIENGESELLSCHAFT Brüningstrasse 50 W-6230 Frankfurt/Main 80(DE)**

(72) Erfinder: **Kibat, Paul-Gerhard, Dr. Gartenstrasse 17 W-6238 Hofheim am Taunus(DE)** Erfinder: **Röthig, Hans-Jürgen, Dr. Am Wäldchen 10 W-6240 Königstein/Taunus(DE)** Erfinder: **Wirth, Klaus Josef, Dr. Johannesallee 28 W-6230 Frankfurt am Main(DE)**

(54) **Schnellwirksame Inhalationsarzneiformen von Diuretika.**

(57) Es wird eine inhalativ anzuwendende Arzneiform enthaltend ein Schleifendiuretikum, das eine mittlere Teilchengröße von etwa 6 bis 1 μm aufweist, beschrieben. Pro Sprühstoß können etwa 1 bis 30 mg Schleifendiuretikum inhaliert werden.

EP 0 465 841 A2

Die Erfindung betrifft inhalativ zu verabreichende Arzneiformen von Schleifendiuretika. Die erfindungsgemäßen Arzneiformen zeichnen sich durch schnellen Wirkungseintritt und durch eine Wirksamkeit im niedrigen therapeutischen Dosisbereich aus. Sie eignen sich besonders zur akuten Medikation z.B. von Lungen-, Herz-, Leber- und Hirnödemen oder von Vergiftungen. Sie stellen eine Alternative zu bekannten parenteralen Injektionszubereitungen von Schleifendiuretika dar, besitzen aber den Vorteil, daß für die eigentliche Verabreichung kein besonders ausgebildetes Fachpersonal erforderlich ist.

Schleifendiuretika wie z.B. Furosemid oder Piretanid werden seit langem erfolgreich angewendet bei Bluthochdruck, Pre-Eklamsie, Nierenerkrankungen, Vergiftungen und Leber-, Hirn- oder Verbrennungsödemen. Üblicherweise wird der Wirkstoff in Form von Tabletten, Retardtabletten, Kapseln, Injektionslösungen, Tropfen oder Infusionen verabreicht.

Nach oraler Gabe tritt die Wirkung nach etwa 0,5 - 1 h ein. Die Bioverfügbarkeit ist abhängig vom Wirkstoff und beträgt z.B. 60 - 70 % bei Furosemid oder 80 - 85 % bei Piretanid. Die therapeutisch wirksame Dosis ist etwa 40 mg bei peroraler Gabe von Furosemid und 3 - 6 mg bei Piretanid.

Über die inhalative Applikation von Diuretika wurde bisher nur wenig berichtet. Mentz et al. (Am. Rev. Respir. Dis. 143, S. 938-943 (1986)) fanden eine schnelle Absorbtion von inhalativ verabreichtem Amilorid.

Nach Untersuchungen von Bianco et al. (The Lancet, S. 252-255, 1988) zeigt Furosemid einen deutlich asthmaprotektiven Effekt, wenn es als Lösung mit einem Vernebler inhalativ verabreicht wird.

Überraschenderweise zeigte sich nun, daß es nach Inhalation feststoffhaltiger Zubereitungen von Schleifendiuretika im Vergleich zur Inhalation einer vernebelten Lösung zu einer rasch einsetzenden Diurese kam.

Inhalativ anzuwendende Arzneiformen bestehen in der Regel aus einer Zubereitung in der der Arzneistoff gelöst oder suspendiert ist oder als Pulver vorliegt und einem Behältnis, das die Ausbringung des Arzneistoffes als feinstverteiltes Aerosol ermöglicht. In der Therapie von Asthma müssen lungengängige Arzneimittel angewendet werden, d.h. Arzneimittel, die beim Einatmen bis in den Bereich der Bronchiolen gelangen können. Dazu ist es nötig, daß die Teilchengröße des zu inhalierenden Arzneimittels überwiegend im Bereich von 1 - 6 $\mu$m liegt.

Verbreitete Anwendung finden treibmittelhaltige Dosieraerosole in Druckgaspackungen, die einen Arzneistoff geeigneter Teilchengröße mit einem Netzmittel, wie z.B. Lecithin, Sorbitantrioleat oder Ölsäure, in einem Treibmittelgemisch suspendiert enthalten (z.B. EP-A-0 162 556). Beim Auslösen eines Dosierventils wird eine Arzneistoffdosis unter Druck freigesetzt und zerstäubt; nach dem schnellen Verdampfen des Treibmittels bleibt ein feinstverteiltes Aerosol von Arzneistoffteilchen zurück, das inhaliert wird.

Treibmittel sind z.B. Gemische von Trichlormonofluormethan (F11) und/oder Dichlortetrafluorethan (F114) und Dichlordifluormethan (F12).

Weiterhin gebräuchlich sind Pulver-Inhalationen, die aus einem Arzneistoffpulver mit mikronisiertem Wirkstoff mit (vergl. z.B. US-Patentschrift 3 957 965) oder ohne (EP-A-0 072 046) Hilfsstoff und einem geeigneten Inhalationsgerät (z.B. Schwedische Patentanmeldung 8501806) bestehen.

Außerdem finden Vernebler zur Inhalation von wäßrigen Lösungen oder Suspensionen weitverbreitete Anwendung (Köhler, D., Med. Mo. Pharm 12, S. 356-361, 1989). Sie sind jedoch in der Notfalltherapie nicht einsetzbar.

Die Erfindung betrifft daher eine inhalativ anzuwendende Arzneiform bestehend aus einem pulverförmigen Arzneistoff oder einem pulverförmigen Gemisch aus Arzneistoff und üblichen Hilfstoffen und einem Behältnis, das die Ausbringung des Arzneistoffs als feinstverteiltes Aerosol ermöglicht, dadurch gekennzeichnet, daß der Arzneistoff ein Schleifendiuretikum ist und eine mittlere Teilchengröße von etwa 6 bis 1 $\mu$m aufweist und pro Sprühstoß etwa 1 bis 30 mg Schleifendiuretikum inhaliert werden können.

Bevorzugte Schleifendiuretika sind Furosemid und Piretanid bzw. deren physiologisch verträgliche Salze.

Im Falle von Furosemid oder einem physiologisch verträglichen Furosemid-Salz können pro Sprühstoß vorzugsweise 5 bis 20 mg inhaliert werden.

Die mittlere Teilchengröße beträgt vorzugsweise etwa 2 bis 4 $\mu$m. Die Arzneiform ist vorzugsweise eine Dosieraerosol oder ein Pulver-Inhalat, das in Kapseln abgefüllt mit Hilfe eines geeigneten Dosiergeräts (handelsüblicher Pulverinhalator) inhaliert wird.

Die erfindungsgemäßen Zubereitungsformen enthalten außer dem Wirkstoff gegebenenfalls noch Hilfsstoffe.

Die Schleifendiuretika liegen vorzugsweise im Gemisch mit üblichen Hilfsstoffen vor. Im Falle von Dosieraerosolen wird z.B. ein Netzmittel zugesetzt. Pulver-Inhalate enthalten als Hilfstoffe z.B. Laktose, Sorbit oder Mannit, wobei der Wirkstoffgehalt vorzugsweise zwischen 20 % und 95 % liegt.

Bei den erfindungsgemäßen Inhalationszubereitungen ist die Wirkstoffmenge pro Sprühstoß/Dosis im Vergleich zu Medikamenten für die Asthma-Therapie wesentlich höher. Wie in der Literatur beschrieben (vergl. z.B. S.W. Hallworth in Drug delivery to the respiratory tract, Herausgeber D. Ganderton und T. Jones, Weinheim-New York, 1987, Kapitel 9, S. 98 ff.) ergeben sich besonders für Dosieraerosole Schwierigkeiten bei der Formulierung derartig hochdosierter Zubereitungen.

Die Erfindung betrifft ferner ein Verfahren zur Herstellung einer inhalativ anzuwendenden Arzneiform bestehend aus einem pulverförmigen Arzneistoff, gegebenenfalls im Gemisch mit üblichen Hilfsstoffen, und einem Behältnis, das die Ausbringung des Arzneistoffs als feinverteiltes Aerosol ermöglicht, dadurch gekennzeichnet, daß man ein Schleifendiuretikum auf eine mittlere Teilchengröße von etwa 6 bis 1 $\mu$m bringt und gegebenenfalls nach Zumischen von üblichen Hilfsstoffen in ein geeignetes Behältnis überführt.

Durch geeignete Wahl der Herstellungstechnologie und des Applikationssystems ist es möglich, geeignete Inhalationszubereitungen mit hohem Wirkstoffgehalt zur Verfügung zu stellen.

Zur Herstellung der erfindungsgemäßen Inhalationsarzneimittel wird der Arzneistoff zunächst durch Mahlung (z.B. mittels Luftstrahlmühle), Sprühtrocknung oder kontrollierte Kristallisation auf eine geeignete Teilchengröße eingestellt.

Durch Verwendung von geeigneten Carriern, die Anwendung einer Sprühtrocknungstechnik oder durch Spheronisation des Wirkstoffpulvers unter Verwendung geringer Hilfsstoffmengen werden Zusammensetzungen für die Inhalation von Pulvern mit den erforderlich hohen Dosen erhalten.

Bei der Herstellung eines Dosieraerosols wird daher der Arzneistoff z.B. mit einem Netzmittel wie Lecithin, Sorbitantrioleat oder Ölsäure versetzt und mit einem üblichen Treibmittelgemisch in einer Druckgaspackung mit Dosierventil befüllt. Ein Pulver-Inhalat läßt sich herstellen, wenn der Arzneistoff z.B. mit Laktose, Sorbit oder Mannit gemischt und in ein geeignetes Dosiergerät oder in Kapseln abgefüllt wird. Alternativ kann auch eine Spheronisation in Gegenwart von z.B. Wasser oder eine Sprühtrocknung mit Hilfsstoffen angewendet werden um freifließende, gut dosierbare Pulver herzustellen, die beim Inhalieren mit einem handelsüblichen Pulverinhalator zu einem feinstverteilten Staub zerfallen.

Die Arzneiform wird so hergestellt, daß pro Sprühstoß etwa 1-30 mg Schleifendiuretikum inhaliert werden können. Der diuretische Effekt von Furosemid tritt im Dosisbereich ab 10 mg auf und führt zu einer maximalen Diurese in der 1. - 2. Stunde nach Inhalation. Die Wirkung ist im Bereich von 10-40 mg dosisabhängig (Tab. 1). Im Vergleich zur peroralen Gabe (Benet, L.Z., J. Pharmacokin. Biopharm. 7, S. 1-27 (1979)) tritt die Diurese schon im Dosisbereich unter 40 mg auf.

Die erfindungsgemäße Arzneizubereitung weist gegenüber der von Bianco et al. in The Lancet, S. 252-255 (1988) beschriebenen Zubereitung folgende Unterschiede/Vorteile auf:

1. Es wird ein Pulver inhaliert und keine Lösung des Schleifendiuretikums.

2. Es handelt sich um eine schnell wirksame Zubereitung, denn die gesamte Wirkstoffdosis wird auf einmal appliziert, während die Inhalation gemäß Stand der Technik über einen Zeitraum von etwa 20 Minuten erfolgt.

3. Die Zubereitung eignet sich zur Ödembehandlung, vorzugsweise zur Lungenödembehandlung, wobei die Inhalation durch den Mund stattfindet.

4. Die Inhalation erfolgt mit Hilfe eines kleinen, tragbaren Gerätes (Inhalator). Die Verneblung der Lösung ist an ein in der Regel nicht in vergleichbarer Art tragbares und überall einsetzbares Gerät gebunden.

Die erfindungsgemäße Zubereitungen besitzen gegenüber den Injektionszubereitungen von Schleifendiuretika den Vorteil, daß für ihre Applikation kein besonders geschultes Personal nötig ist.

**Beispiel 1**

Furosemid wird mit einer Luftstrahlmühle auf eine mittlere Teilchengröße von 2 $\mu$m mikronisiert. In eine Aerosoldose werden 400 mg des mikronisierten Wirkstoffes und 25 mg Sorbitantrioleat gegeben, dann wird ein Dosierventil (100 $\mu$l) aufgecrimpt. Mit einer Druckfüllanlage werden 5,095 g Treibmittelgemisch (Treibmittel F12:F114 im Verhältnis 40:60) über das Ventil eingefüllt. Die fertige Zubereitung wird mit einem geeigneten Applikator versehen. Ein Sprühstoß enthält 10 mg Furosemid.

**Beispiel 2**

Piretanid wird mit einer Luftstrahlmühle auf eine mittlere Teilchengröße von 2 $\mu$m mikronisiert. In eine Aerosoldose werden 250 mg des mikronisierten Wirkstoffes und 20 mg Sorbitantrioleat gegeben, dann wird ein Dosierventil (100 $\mu$l) aufgecrimpt. Mit einer Druckfüllanlage werden 10,0 g Treibmittelgemisch (Treibmittel F11:F12:F114 im Verhältnis 25:50:25) über das Ventil eingefüllt. Die fertige Zubereitung wird mit einem geeigneten Applikator versehen. Ein Sprühstoß enthält 3,4 mg Piretanid.

**Beispiel 3**

Mikronisiertes Furosemid (mittlere Teilchengröße 2,0 μm) wird im Verhältnis 1:1 mit Laktose (Teilchengröße 30-80 μm) vermischt. 20 mg der Mischung werden in Hartgelatinekapseln Gr. 5 abgefüllt; die Zubereitung kann mit einem handelsüblichen Inhalator inhaliert werden.

**Beispiel 4**

Furosemid wird mit 1N Natronlauge als Na-Salz gelöst und die Lösung auf pH 7,4 eingestellt. Die Lösung wird mit einem Büchi-Mini-Sprayer 190 bei 100-140°C versprüht. Das sprühgetrocknete Produkt hat eine mittlere Teilchengröße von 3 μm und ist freifließend. 10 mg des sprühgetrockneten Na-Salzes werden in Kapseln abgefüllt und mittels handelsüblicher Inhalatoren inhaliert.

**Beispiel 5**

Mikronisiertes Piretanid (mittlere Teilchengröße 2 μm) wird mit Glukose im Verhältnis 1:1 vermischt. Das Gemisch wird einer Spheronisation unter Zusatz von 10 % (v/v) Wasser unterworfen. Die gebildeten Agglomerate werden durch Siebung auf eine Teilchengröße von 100-300 μm eingestellt und in Kapseln (5 mg/Kapseln entspr. 2,5 mg Piretamid) abgefüllt, um mit handelsüblichen Inhalatoren inhaliert werden zu können (wobei die Agglomerate wieder in Einzelteile zerfallen).

**Beispiel 6**

Die Zubereitung aus Beispiel 1 wird an 6 weiblichen Probandinnen in verschiedenen Dosierungen (10-40 mg) im cross-over Test gegen Placebo getestet. Steigende Dosen werden appliziert, indem das Dosieraerosol 1-, 2- oder 3-mal ausgelöst und inhaliert wird. Tab. 1 zeigt die Ergebnisse der Urinausscheidung in verschiedenen Zeitintervallen.

Tabelle 1

| DOSIS | URINVOLUMEN (ML/ZEIT) | | | |
|---|---|---|---|---|
| | -2 - 0 H | 0 - 2 H | 2 - 4 H | 4 - 8 H |
| PLACEBO | 103 ($\pm$ 55) | 165 ($\pm$ 148) | 93 ($\pm$ 34) | 337 ($\pm$ 354) |
| 10 MG | 89 ($\pm$ 68) | 492 ($\pm$ 252) | 104 ($\pm$ 37) | 317 ($\pm$ 216) |
| 20 MG | 61 ($\pm$ 39) | 724 ($\pm$ 198) | 259 ($\pm$ 129) | 445 ($\pm$ 208) |
| 40 MG | 81 ($\pm$ 46) | 1103 ($\pm$ 321) | 264 ($\pm$ 265) | 249 ($\pm$ 133) |

**Patentansprüche**

1. Inhalativ anzuwendende Arzneiform bestehend aus einem pulverförmigen Arzneistoff oder einem pulverförmigen Gemisch aus Arzneistoff und üblichen Hilfstoffen und einem Behältnis, das die Ausbringung des Arzneistoffs als feinstverteiltes Aerosol ermöglicht, dadurch gekennzeichnet, daß der Arzneistoff ein Schleifendiuretikum ist und eine mittlere Teilchengröße von etwa 6 bis 1 µm aufweist und pro Sprühstoß etwa 1 bis 30 mg Schleifendiuretikum inhaliert werden können.

2. Arzneiform gemäß Anspruch 1, dadurch gekennzeichnet, daß sie als Schleifendiuretikum Furosemid oder Piretanid bzw. deren physiologisch verträgliche Salze enthält.

3. Verfahren zur Herstellung einer inhalativ anzuwendenden Arzneiform bestehend aus einem pulverförmigen Arzneistoff, gegebenenfalls im Gemisch mit üblichen Hilfsstoffen, und einem Behältnis, das die Ausbringung des Arzneistoffs als feinverteiltes Aerosol ermöglicht, dadurch gekennzeichnet, daß man ein Schleifendiuretikum auf eine mittlere Teilchengröße von etwa 6 bis 1 µm bringt und gegebenenfalls nach Zumischen von üblichen Hilfstoffen in ein geeignetes Behältnis überführt.

**Patentansprüche für folgende Vertragsstaaten: ES, GR**

1. Verfahren zur Herstellung einer inhalativ anzuwendenden Arzneiform bestehend aus einem pulverförmigen Arzneistoff, gegebenenfalls im Gemisch mit üblichen Hilfsstoffen, und einem Behältnis, das die Ausbringung des Arzneistoffs als feinverteiltes Aerosol ermöglicht, dadurch gekennzeichnet, daß man ein Schleifendiuretikum auf eine mittlere Teilchengröße von etwa 6 bis 1 µm bringt und gegebenenfalls nach Zumischen von üblichen Hilfstoffen in ein geeignetes Behältnis überführt, so daß pro Sprühstoß etwa 1 bis 30 mg Schleifendiuretikum inhaliert werden können.

2. Verfahren gemäß Anspruch 1, dadurch gekennzeichnet, daß als Schleifendiuretikum Furosemid oder Piretanid bzw. deren physiologisch verträgliche Salze eingesetzt werden.